# EUROPÄISCHE PATENTSCHRIFT

(11) **EP 0 535 060 B1**
(45) Veröffentlichungstag und Bekanntmachung des Hinweises auf die Patenterteilung: **22.02.1995**
(21) Anmeldenummer: 91911239.1
(22) Anmeldetag: 12.06.1991
(51) Int. Cl.: C07K 14/37, A01N 63/04

(54) **ANTIFUNGISCHES POLYPEPTID, VERFAHREN ZU SEINER HERSTELLUNG**
ANTIFUNGAL POLYPEPTIDE, PROCESS FOR PRODUCING IT
POLYPEPTIDE FONGICIDE ET SON PROCEDE DE PRODUCTION

(30) Priorität: 15.06.1990 DE 4019105; 06.03.1991 DE 4107140
(43) Veröffentlichungstag der Anmeldung: 07.04.1993
(73) Patentinhaber: Hoechst Schering AgrEvo GmbH, 13509 Berlin (DE)
(72) Erfinder: ULBRICH, Norbert, D-1000 Berlin 38 (DE); HILGENFELD, Rolf, D-6382 Friedrichsdorf 2 (DE); HÄNEL, Heinz, D-6370 Oberursel 6 (DE); SACHSE, Burkhard, D-6233 Kelkheim (DE); BRAUN, Peter, D-6500 Mainz 32 (DE); WINK, Joachim, D-6074 Rödermark (DE); ECKES, Peter, D-6233 Kelkheim (DE); LOGEMANN, Jürgen, D-5000 Köln 30 (DE); SCHELL, Jozef, D-5000 Köln 30 (DE)
(86) Internationale Anmeldenummer: EP9101094
(87) Internationale Veröffentlichungsnummer: WO9119738

(56) Entgegenhaltungen:
- EP-A- 320 130
- WO-A-88/00976
- WO-A-89/04371
- APPLIED MICROBIOLOGY, vol. 13, No.3, May 1965, pages 314-321; OLSON, B.H., et al.: "Alpha sarcin, a new antitumor agent" (cited in the application) , see page 316, left-hand column, line 41- line 51 see page 317, right -hand column, line 21 - line 29 see page 320, left-hand column, line 7-line 10;table 3
- NUCLEIC ACIDS RESEARCH, vol. 18, No. 1990, ARLINGTON, VIRGINIA US page 3987, WNENDT; S., et al.: "Cloning and nucleotide sequence of a cDNA encoding the antifungal-protein of Aspergillus giganteus and preliminary characterization of the native gene" see the whole document
- EUR.J.BIOCHEM, vol. 193, No. 1, 1990, pages 31-38; NAKAYA, K.,ET.AL.: "Amino acid sequence and disulfide bridges of an antifugal-protein isolated from Aspergillus giganteus" see the whole document
- J. CELL. BIOCHEM.SUPPL, vol. 14E, 1990 & SYMPOSIUM APRIL 16-22, 1990, page 268; BROGLIE, R., ET AL.:"Chitinase expression in transgenic plants: increased protection against a soil-borne fungal pathogen"

## Beschreibung

Antifungische Polypeptide, z.B. Lectine, konnten aus monokotyledonen bzw. dicotyledonen Pflanzen isoliert werden [Ramshaw, J.A.M. (1982) in Nucleic Acids and Proteins in Plants I. Encyclopedia of Plant Physiology, New Series, (Boulter, D. and Parthier, B. eds.) Vol. 14 A, pp. 229-279, Springer, Berlin]. Ferner wurde beobachtet, daß die Biosynthese von Thionin durch Befall der Pflanze mit pathogenen Pilzen ausgelöst wird. Daher nimmt man an, daß dieses Protein antifungische Aktivität besitzt [Bohlmann, H. et al. (1989) EMBO J. 7, 1559-1565, Broekaert, W.F. et al. (1989) Science 245, 1100-1102].

Antifungische Polypeptide konnten ebenfalls aus Mikroorganismen isoliert werden. In der Fermentationsbrühe von Aspergillus giganteus wurde z.B. ein ähnliches Protein detektiert (Olson, B.M. & Goerner, G.L. (1965) Appl. Microbiol. 13, 314-321), welches jedoch im Vergleich zum erfindungsgemäßen Protein eine um zwei Lysinreste verkürzte Aminosäuresequenz besitzt.

Wir haben nun ein Polypeptid mit antifungischer Wirkung aus Aspergillus giganteus isolieren und seine Sequenz bestimmen können.

Die Nukleotidsequenz für das in der Hauptanmeldung beanspruchte Peptid wurde von Wnendt et al. (Nucl. Acids Res. 18, S. 3987, September 1990) publiziert.

Wir haben außerdem gefunden, daß durch Expression des Gens, welches für da in der o.g. Hauptanmeldung beanspruchte Polypeptid kodiert, in Pflanzen eine starke Hemmung des Wachstums von phytophathogenen Pilzen auf der Pflanze erreicht wird.

Die Erfindung betrifft somit:
Ein Polypeptid mit der Aminosäuresequenz 
Ala-Thr-Tyr-Asn-Gly-Lys-Cys-Tyr-Lys-Lys-Asp-Asn-Ile-
Cys-Lys-Tyr-Lys-Ala-Gln-Ser-Gly-Lys-Thr-Ala-Ile-Cys-
Lys-Cys-Tyr-Val-Lys-Lys-Cys-Pro-Arg-Asp-Gly-Ala-Lys-
Cys-Glu-Phe-Asp-Ser-Tyr-Lys-Gly-Lys-Cys-Tyr-Cys.
Ein Verfahren zur Herstellung des unter 1) charakterisierten Polypeptids, das dadurch gekennzeichnet ist, daß Aspergillus giganteus kultiviert wird und das Polypeptid isoliert wird.

Verwendung des Polypeptids als antifungisches Agens
Eine Pflanzenzelle, die das Gen des antifungischen Peptids aus Aspergillus giganteus exprimiert.

Pflanze, enthaltend Zellen, nach Anspruch 1.

Eine Pflanze, pflanzliches Gewebe oder Vermehrungsgut, gezüchtet aus einer Pflanzenzelle nach Anspruch 1.

Ein Verfahren zur Herstellung der Pflanzenzelle dadurch gekennzeichnet, daß das Gen des antifungischen Polypeptids aus Aspergillus giganteus in der Pflanzenzelle exprimiert wird.

Im folgenden wird die Erfindung detalliert beschrieben, insbesondere in ihren bevorzugten Ausführungsformen. Außerdem wird die Erfindung durch den Inhalt der Ansprüche bestimmt.

Das erfindungsgemäße Polypeptid wird von Aspergillus giganteus bei aerober Kultivierung synthetisiert. Als bevorzugte Kohlenstoffquelle eignen sich assimilierbare Kohlenhydrate und Zuckeralkohole, wie Fructose, Lactose oder Mannit, sowie kohlenhydrathaltige Naturprodukte. Als bevorzugte stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. Außerdem kann die Nährlösung beispielsweise Chloride, Carbonate, Sulfate oder Phosphate der Alkali- oder Erdalkalimetalle, Eisen, Zink und Mangan als zusätzliche anorganische Salze enthalten.

Die Bildung der Verbindung des erfindungsgemäßen Polypeptids verläuft besonders gut in einer Nährlösung, die 0,5 bis 2 % Fleischextrakte, 0,5 bis 2 % Pepton, 0,5 bis 4 % Stärke sowie 0,1 bis 1 % Kochsalz enthält, jeweils bezogen auf das Gewicht der gesamten Nährlösung.

Die Fermentation erfolgt aerob, also beispielsweise submers unter Schütteln oder Rühren in Schüttelkolben oder Fermentern, gegebenenfalls unter Einführen von Luft oder Sauerstoff. Sie kann in einem Temperaturbereich von etwa 18 bis 35°C, vorzugsweise bei etwa 25 bis 30°C erfolgen. Man kultiviert den Mikroorganismus unter den genannten Bedingungen bis die stationäre Phase erreicht ist, etwa 70 bis 80 Stunden.

Vorteilhaft kultiviert man in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1:10, überimpft werden. Die Vorkultur erhält man beispielsweise, indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Malz-Agar, wachsen läßt.

Der Fermentationsverlauf kann anhand des pH-Wertes der Kultur oder des Mycelvolumens, durch Dünnschichtchromatographie, Ausprüfen der biologischen Aktivität oder immunologisch überwacht werden.

Die Isolierung des Polypeptids aus dem Kulturmedium erfolgt nach bekannten Methoden unter Berücksichtigung der chemischen, physikalischen und biologischen Eigenschaften der Produkte. Das Polypeptid liegt in der Kulturbrühe vor.

Die Brühe wird vom Mycel befreit und anschließend an einem Kationenaustauscher chromatographiert. Nach Dialyse und Aufkonzentration wird eine Gelfiltration, bevorzugt an einem Agaroseacrylamidcopolymer, durchgeführt. Die anschließende, zur Sequenzanalyse notwendige Feinreinigung des Polypeptids kann mit HPLC mit Hilfe eines linearen Gradienten auf einer Säule mit hydrophober Wechselwirkung durchgeführt werden.

Die enzymatische Verdauung des Peptids zur Aufklärung der Aminosäuresequenz wird nach an sich bekannten Methoden durchgeführt und resultiert in der oben aufgeführten Aminosäuresequenz. Charakteristisch für das Enzym ist dessen Thermostabilität. Nach Erhitzen auf 100°C über einen Zeitraum von 20 Minuten zeigt es keinen Aktivitätsverlust.

Das Protein kann als antifungisches Agens, insbesondere gegen phytopathogene Pilze eingesetzt werden. Die Erfindung betrifft daher auch ein Mittel zur Inhibierung oder Abtötung von phytopathogenen Pilzen auf Pflanzen, das durch einen Gehalt an erfindungsgemäßem Protein gekennzeichnet ist. Phytopathogene Krankheitserreger lassen sich erfolgreich protektiv und kurativ bekämpfen. Das Wirkungsspektrum der beanspruchten Verbindung erfaßt eine Vielzahl verschiedener wirtschaftlich bedeutender phytopathogener Pilze, wie z. B. Piricularia oryzae, Venturia inaegualis, Cercospora beticola, Echte Mehltauarten, Fusariumarten, Plasmopora viticola, Pseudoperonospora cubensis, verschiedene Rostpilze und Pseudocercosporella herpotrichoides. Besonders gut werden Benzimidazol- und Dicarboximid-sensitive und resistente Botrytis cinerea-Stämme erfaßt.

Die erfindungsgemäße Verbindung eignet sich daneben auch für den Einsatz in technischen Bereichen, beispielsweise als Konservierungsmittel für technische Produkte und Nahrungs- bzw. Futtermittel.

Gegenstand der Erfindung sind auch Mittel, die die o.g. Verbindung neben geeigneten Formulierungshilfsmitteln enthalten.

Die einzelnen Formulierungstypen sind dem Fachmann bekannt und werden beispielsweise beschrieben in: Winnacker-Küchler, "Chemische Technologie", Band 7, C. Hanser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in:
Watkins, "Handbook of Insecticide Dust Diluents and Carriers", 2nd Ed., Darland Books, Caldwell N.J.; H.v.Olphen, "Introduction to Clay Colloid Chemistry, 2nd Ed., J. Wiley & Sons, N.Y.; Marschen, "Solvents Guide", 2nd Ed.; Interscience, N.Y. 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix.

Als literaturbekannte Fungizide, die erfindungsgemäß mit der Verbindung der Formel I kombiniert werden können, sind z.B. folgende Produkte zu nennen:
Anilazine, Benalaxyl, Benodanil, Benomyl, Binapacryl, Bitertanol, Buthiobat, Captafol, Captan, Carbendazim, Carboxin, CGD-94240 F, Chlobenzthiazone, Chlorthalonil, Cymoxanil, Cyproconazole, Cyprofuram, Dichlofluanid, Dichlomezin, Diclobutrazol, Diethofencarb, Difluconazole, Dimethirimol, Dimethomorph, Diniconazole, Dinocap, Dithianon, Dodemorph, Dodine, Edifenfos, Ethirimol, Etridiazol, Fenarimol, Fenfuram, Fenpiclonil, Fenpropidin, Fenpropimorph, Fentinacetate, Fentinhydroxide, Fluaziram, Fluobenzimine, Fluorimide, Flusilazole, Flutolanil, Flutriafol, Folpet, Fosetylaluminium, Fuberidazole, Furalaxyl, Furmecyclox, Guazatine, Hexaconazole, Imazalil, Iprobenfos, Iprodione, Isoprothiolane, Kupferverbindungen wie Cu-Oxychlorid, Oxine-Cu, Cu-Oxide, Mancozeb, Maneb, Mepronil, Metalaxyl, Methasulfocarb, Methfuroxam, Myclobutanil, Nabam, Nitrothalidopropyl, Nuarimol, Ofurace, Oxadixyl, Oxycarboxin, Penconazol, Pencycuron, PP 969, Probenazole, Probineb, Prochloraz, Procymidon, Propamocarb, Propiconazol, Prothiocarb, Pyracarbolid, Pyrifenox, Pyroquilon, Rabenzazole, Sulfur, Tebuconazole, Thiabendazole, Thiofanatemethyl, Thiram, Tolclofos-methyl, Tolylfluanid, Triadimefon, Triadimenol, Tricyclazole, Tridemorph, Triflumizol, Triforine, Vinchlozolin, Zineb, Natriumdodecylsulfonat, Natrium-dodecylsulfat, Natrium-C13/C15-alkoholethersulfonat, Natrium-cetostearylphosphateester, Dioctyl-natrium-sulfosuccinat, Natrium-isopropylnaphthalinsulfonat, Natrium-methylen-bisnaphthalinsulfonat, Cetyl-trimethyl-ammoniumchlorid, Salze von langkettigen primären, sekundären oder tertiären Aminen, Alkylpropylenamine, Lauryl-pyrimidiniumbromid, ethoxilierte quarternierte Fettamine, Alkyl-dimethyl-benzyl-ammoniumchlorid und 1-Hydroxyethyl-2-alkyl-imidazolin.

Die obengenannten Kombinationspartner stellen bekannte Wirkstoffe dar, die zum großen Teil in CH.R Worthing, U.S.B. Walker, The Pesticide Manual, 7. Auflage (1983), British Crop Protection Council, beschrieben sind.

Darüber hinaus kann der erfindungsgemäße Wirkstoff in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden oder Herbiziden vorliegen. Zu den Insektiziden zählen beispielsweise Phosphorsäureester, Carbamate, Carbonsäureester, Formamidine, Zinnverbindungen, durch Mikroorganismen hergestellte Stoffe u.a. Bevorzugte Mischungspartner sind:

### 1. Aus der Gruppe der Phosphorverbindungen

Acephate, Azamethiphos, Azinphos-ethyl, Azinphos-methyl, Bromophos, Bromophos-ethyl, Chlorfenvinphos, Chlormephos, Chlorpyrifos, Chlorpyrifos-methyl, Demeton, Demeton-S-methyl, Demeton-S-methyl sulfphone, Dialifos, Diazinon, Dichlorvos, Dicrotophos, 0,0-1,2,2,2-tetrachlorethylphosphorthioate (SD 208 304), Dimethoate, Disulfoton, EPN, Ethion, Ethoprophos, Etrimfos, Famphur, Fenaminphos, Fenitriothion, Fensulfothion, Fenthion, Fonofos, Formothion, Heptenophos, Isozophos, Isothioate, Isoxathion, Malathion, Methacrifos, Methamidophos, Methidation, Salithion, Mevinphos, Monocrotophos, Naled, Omethoate, Oxydemetonmethyl, Parathion, Parathion-methyl, Phenthoate, Phorate, Phosalone, Phosfolan, Phosmet, Phosphamidon, Phoxim, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tetrachlorvinphos, Thiometon, Triazophos, Trichlorphon, Vamidothion;

### 2. Aus der Gruppe der Carbamate

Aldicarb, 2-sec.-Butylphenylmethylcarbamate (BPMC), Carbaryl, Carbofuran, Carbosulfan, Cloethocarb, Benfuracarb, Ethiofencarb, Furathiocarb, Isoprocarb, Methomyl, 5-Methyl-m-cu-menylbutyryl(methyl)carbamate, Oxamyl, Primicarb, Propoxur, Thiodicarb, Thiofanox, Ethyl 4,6,9-triaza-4-benzyl-6,10-dimethyl-8-oxa-7-oxo-5,11-dithia-9-dodecenoate (OK 135), 1-Methylthio(ethylidene-amino)-N-methyl-N-(morpholinothio)carbamate (UC 51717);

### 3. Aus der Gruppe der Carbonsäureester

Allethrin, Alphamethrin, 5-Benzyl-3-furylmethyl-(E)-(1R)-cis-2,2-di-methyl-3-(2-oxothiolan-3-ylidenemethyl)- cyclopropanecarboxylate, Bioallethrin, Bioallethrin(S)-cyclopentylisomer), Bioresmethrin, Biphenate, (RS)-1-Cyano-1- (6-phenoxy-2-pyridyl)-methyl-(1RS)-trans-3-(4-tert.-butylphenyl)-2,2-dimethylcyclopropanecarboxylate (NCI 85193), Cycloprothrin, Cyfluthrin, Cyhalothrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-Isomer), Permethrin, Pheothrin ((R)-Isomer), d-Pralethrin, Pyrethrine (natürliche Produkte), Resmethrin, Tefluthrin, Tetramethrin, Tralomethrin.

### 4. Aus der Gruppe der Amidine

Amitraz, Chlordimeform

### 5. Aus der Gruppe der Zinnverbindungen

Cyhexatin, Fenbutatinoxide

### 6. Sonstige

Abamectin, Bacillus thuringiensis, Bensultap, Binapacyl, Bromopropylate, Buprofezin, Camphechlor, Cartap, Chlorbenzialate, Chlorfluazuron, 2- (4- Chlorphenyl)-4,5-diphenylthiophen (UBI-T 930), Chlofentezine, Cyclopropancarbonsäure(2-naphthylmethyl)-ester (Ro 12-0470), Cyromazin, N-(3,5-Dichlor-4-(1,1,2,3,3,3-hexafluor-1-propyloxy)phenyl)carbamoyl)-2-chlorbenzcarboximid-säureethylester, DDT, Dicofol, N-(N-(3,5-Di-chlor-4-(1,1,2,2-tetrafluorethoxy)phenylamino)-carbonyl)-2,6-difluorbenzamid (XRD 473), Diflubenzuron, N-(2,3-Dihydro-3-methyl-1,3-thiazol-2-ylidene)2,4-xylidine, Dinobuton, Dinocap, Endosulfan, Ethofenprox, (4-Ethoxyphenyl)(dimethyl)(3-(3-phenoxyphenyl)propyl)-silan, (4-Ethoxyphenyl)(3-(4-fluoro-3-phenoxyphenyl) propyl)dimethylsilan, Fenoxycarb, 2-Fluoro-5-(4-(4-ethoxyphenyl)-4-methyl-1-pentyl)diphenylether (MTI 800), Granulose- und Kernpolyederviren, Fenthiocarb, Flubenzimine, Flucycloxuron, Flufenoxuron, Gamma-HCH, Hexythiazox, Hydramethylnon (AC 217 300), Ivermectin, 2-Nitromethyl-4,5-dihydro-6H-thiazin (SD 52618), 2-Nitromethyl-3,4-dihydrothiazol (SD 35651), 2-Nitromethylene-1,2-thiazinan-3-ylcarbamaldehyde (WL 108 477), Propargite, Teflubenzuron, Tetradifon, Tetrasul, Thicyclam, Triflumaron.

Alle gentechnologischen Arbeiten zur Expression des Gens in Pflanzen wurden, wenn nicht anders angegeben, entsprechend den in "Molecular Cloning" (Maniatis et al., Cold. Spring Harbour, 1982) dargestellten Methoden durchgeführt.

Alle verwendeten Enzyme wurden, wenn nicht anders angegeben, von der Firma Boehringer Mannheim bezogen. Die Chemikalien stammen von Merck (Darmstadt).

Die c-DNA-Sequenz des antifungischen Peptids wird für die Klonierung im Vektor mit Enden versehen, die in BamH1 und Sal1 Restriktionsschnittstellen ligiert werden können.

Als Klonierungsvektor wurde pDH 51 (Pietrzak et al., Nucl. Acids Res. 14, S. 5857, 1986) verwendet.

Der Vektor pDH 51 wird mit den Restriktionsenzymen BamH1 und Sal1 zwischen Promotor und Terminator geöffnet. Der Vektor pDH 51 ist ein pUC 18 Derivat, ds Promotor- und Terminator-Sequenzen des 35S Transkripts aus Cauliflower Mosaic Virus erhält. Diese Sequenzen werden vom pflanzlichen Transkriptionsapparat erkannt und führen zu einer starken konstitutiven Expression des mit ihnen verbundenen Gens in Pflanzen.

Außer dem 35S Promotor kann jeder andere, in Pflanzen wirksame Promotor verwendet werden.

Die DNA des antifungischen Peptids wird dann über die BamH1- und Sal1-Schnittstelle in den Vektor kloniert, so daß sich stromaufwärts die Promotor- und stromabwärts die Temrinatorsequenz befindet.

Die Transkriptionseinheit: Promotor, Gen und Terminator wird mit dem Restriktionsenzym EcoRI aus dem Vektor herausgeschnitten und in einen Pflanzentransformationsvektor kloniert.

Als Pflanzentransformationsvektoren können z.B. folgende Vektoren bzw. ihre Derivate verwendet werden:
- pOCA 18 (Olszewski et al., Nucl. Acids Res. 16, S. 10765, 1988) und
- pPCV 310 (Koncz und Schell, MGG 204, S. 383, 1986).

Jeder andere Pflanzentransformationsvektor, welcher die für eine Insertion in das Genom von Pflanzen wichtige Border-Sequenz des Ti-Plasmids von Agrobacterium tumefaciens besitzt, kann ebenfalls eingesetzt werden.

Nachdem Transkriptionseinheit und Vektor über die EcoRI-Schnittstelle ligiert sind, wird das Konstrukt in den Agrobakterien-Stamm MP90RK (Koncz und Schell, MGG 204, S. 383, 1986); oder EHA 101 (Hood et al., J. Bacteriol. 168, S. 1291, 1986) konjugiert.

Die Methode der Konjugation ist in "Plant Molecular Biology/Manual" (Kluwer Academic Publisher, Dordrecht, 1988) beschrieben.

Tabak- und Tomatenpflanzen werden nach der "leaf disc" Methode (Horsch et al. Science 227, 1229, (1985) transformiert. Prinzipiell kann auch jede andere dikotyledone Pflanze transformiert werden. Transformierte Sprosse werden aufgrund der mitübertragenen Resistenz gegen das Antibiotikum Kanamycin selektioniert. Durch DNA-Analyse (Southern Blotting), RNA Analyse (Northern Blotting) und Protein-Analyse mit spezifischen Antikörpern (Western Blotting) wird die Expression des antifungischen Proteins in den transformierten Nutzpflanzen überprüft.

Monokotyledone Pflanzen werden mittels direktem Gentransfer in Protoplasten transformiert. Diese Protoplasten werden anschließend zu intakten Pflanzen regeneriert (Gene Transfer in Cerials, Potrykus I., Biotechnology 8, S. 535, 1990).

Pflanzen, die das Protein exprimieren, werden in das Gewächshaus überführt und mit pytophatogenen Pilzen inokuliert. Abhängig von der Art der Pilze erfolgt die Inokulation mit in Suspension befindlichen Hyphen oder Konidiosporen. Das für die Inokulation zu verwendende Blattstadium der Pflanze, sowie die Inkubationsdauer ist abhängig von der Pflanze und dem pythopathogenen Pilz.

Im folgenden wird die Erfindung anhand von Beispielen weitergehend erläutert.

### Beispiele

### 1. Kultivierung von Aspergillus giganteus

a) Herstellung einer Sporensuspension des Produzentenstamms: 100 ml Nährlösung (0,75 % Fleischextrakt, 1 % Pepton, 2 % lösliche Stärke und 0,5 % NaCl) in einem 500 ml Erlenmeyerkolben werden mit dem Stamm beimpft und 72 Stunden bei 25°C und 120 UpM auf der rotierenden Schüttelmaschine inkubiert. Anschließend werden 20 ml Kulturflüssigkeit in einem 500 ml Erlenmeyerkolben mit dem Nährboden der oben genannten Zusammensetzung, dem 20 g Agar/l zur Verfestigung zugegeben wurde, gleichmäßig verteilt und dekantiert. Die Kulturen werden 10 bis 14 Tage bei 25°C inkubiert. Die nach dieser Zeit entstandenen Sporen eines Kolbens werden mit 500 ml entionisiertem Wasser, das einen Tropfen eines handelsüblichen nichtionischen Tensids enthält, abgeschwemmt, sofort weiterverwendet oder bei -22°C aufbewahrt.
b) Herstellung einer Kultur bzw. Vorkultur des Produzentenstammes im Erlenmeyerkolben:
   Ein 500 ml Erlenmeyerkolben mit 100 ml einer Nährlösung der obengenannten Zusammensetzung (pH 6,5) wird mit einer auf einem Schrägröhrchen gezogenen Kultur oder mit 0,2 ml Sporensuspension angeimpft und auf einer Schüttelmaschine bei 120 UpM und 25°C inkubiert. Das Produktionsmaximum wird nach ca. 80 Stunden (pH = 5,3) erreicht. Die Ausbeuten liegen bei ca. 1 bis 2 mg/l.

### 2. Isolierung und Reinigung des Polypeptids

Das Polypeptid wird gemäß (Olson, B.M. & Goerner, G.L. (1965) Appl. Microbiol. 13, 314-321) aufgearbeitet. Die anschließende Feinreinigung wird mit Hilfe der HPLC auf einer ODS-120T-Säule (0,46 x 25 cm) mit einem linearen Gradienten von 5 bis 20 % Acetonitril (v/v), der mit 0,1 % Trifluoressigsäure versetzt ist, bei einer Durchflußrate von 2 ml/min durchgeführt.

### 3. Charakterisierung des Polypeptids

a) Enzymatische Spaltung:
   Zur enzymatischen Spaltung wird das Polypeptid in 1 M Tris-HCL Puffer (pH 8), dem 6 M Guanidin und 2 mM EDTA zugesetzt wird, bei 37°C über einen Zeitraum von 2 Stunden inkubiert und durch Zugabe von 0,1 M Dithiotritol bei 37°C über einen Zeitraum von 12 Stunden reduziert. Das reduzierte Protein wird dann mit 0,15 M Iodacetamid bei 37°C 20 Minuten behandelt. Das S-carboxyamidomethylierte Protein wird wie oben beschrieben per HPLC von Verunreinigungen abgetrennt und dann mit Chymotrypsin (EC 3.4.21.1) in 0,1 M Ammoniumbicarbonat-Puffer (pH 8) bei 37°C 30 Minuten behandelt.
   Die Protein-Konzentration beträgt 150 »g/ml bei einem Enzym:Substrat Verhältnis von 1:50 (w/w).
   Die Verdauung mit Staphylococcus aureus V 8 Protease (EC 3.4.21.15) wird in 0,1 M Bicarbonat Puffer (pH 8) 18 Stunden bei 37°C durchgeführt. Das Enzym:Substrat-Verhältnis beträgt dabei 1:30 (w/w).
   Die Behandlung des nativen oder S-carboxymethylierten Proteins mit Lysylendopeptidase (EC 3.4.21.50) erfolgt durch Inkubation in 8 M Harnstoff, dem 0,1 M Tris-HGl (pH 9) zugesetzt wird, 30 Minuten bei 37°C mit anschließender Verdauung in 0,1 M Tris-HGl (pH 8) 12 Stunden bei 37°C, bei einem Enzym:Substrat-Verhältnis von 1:100 (w/w).
   Die Verdauung des S-carboxyamidomethylierten Proteins mit Carboxypeptidase Y (EC 3.4.16.1) wird in 25 mM Phosphat Puffer (pH 6,5) bei 37°C bis zu 4 Stunden durchgeführt. Hierbei beträgt das Enzym:Substrat-Verhältnis 1:3 (w/w).
b) Aminosäureanalyse und Sequenzabstimmung:
   Das Protein und die Bruchstücke werden in 6 N HCl 22 Stunden bei 110°C hydrolysiert. Die Aminosäuresequenzanalyse wird mit Applied Biosystems Protein-Sequencer (Modell 470 A) durchgeführt.
   Phenylhydantoisaminosäurederivate werden mit HPLC (ODS-120 T, 0,46 x 25 cm) gemäß Tsunasawa, S. et al. (1985) J. Biochem. (Tokyo) 97, 701-704, ausgeführt. Phenylhydantoin Cam-Cys wird, wie in Theerasilp, S. et al. (1989), J. Biol. Chem. 264, 6655-6659 beschrieben, bestimmt.
c) Bestimmung von Cystin- und Cystein-Bruchstücken Die Analyse wird durch Reaktion mit Ellman's Reagens, 5,5'-Dithiobis-(2 nitrobenzoesäure), vor und nach Behandlung mit Natriumborhydrid, entsprechend Henschen, A. (1986) in Advanced Method in Protein Microsequence Analysis (Wittmann-Liebold, B, Salnikow, J. & Erdmann, V.A., eds) pp. 244-255, Springer-Verlag, Berlin, durchgeführt. Das Protein wird in Gegenwart oder Abwesenheit von 2,5 % Natriumborhydrid in Wasser 1 Stunde bei 50°C inkubiert. Nach anschließender Zerstörung des Borhydrids durch Behandlung mit 0,3 N HCl für 30 Minuten, wird die Probe mit 0,11 % Ellman's Reagenz in 0,04 M Phosphat Puffer (pH 8,1) 5 Minuten inkubiert. Es wird dann die Absorption bei 412 nm gemessen.

### Ergebnis:

Das erfindungsgemäße Protein zeigt die folgende Aminosäuresequenz: 
Ala-Thr-Tyr-Asn-Gly-Lys-Cys-Tyr-Lys-Lys-Asp-Asn-Ile-
Cys-Lys-Tyr-Lys-Ala-Gln-Ser-Gly-Lys-Thr-Ala-Ile-Cys-
Lys-Cys-Tyr-Val-Lys-Lys-Cys-Pro-Arg-Asp-Gly-Ala-Lys-
Cys-Glu-Phe-Asp-Ser-Tyr-Lys-Gly-Lys-Cys-Tyr-Cys.
Die Summe der Molekulargewichte der analysierten Aminosäuren beträgt 5788 Dalton.

4 Disulfid Brücken können wie folgt identifiziert werden:

Cys 7-Cys 33, Cys 14-Cys 40, Cys 26-Cys 28 und Cys 49-
Cys 51

7 9
L4 Cys-Tyr-Lys
Cys-Pro-Arg-Asp-Gly-Ala-Lys
33 39

23 27
L9 Thr-Ala-Ile-Cys-Lys
Cys-Tyr-Val-Lys
28 31

11 15
L10 Asp-Asn-Ile-Cys-Lys
Cys-Glu-Phe-Asp-Ser-Tyr-Lys
40 46

49 51
L8 Cys-Tyr-Cys

### 4. Antifungische Aktivität

Die antifungische Aktivität wird gemäß Roether W. et al., Mykosen 27 (1) 14-28, (1984), durchgeführt. Die Ergebnisse sind in der folgenden Tabelle 1 aufgeführt.

**Tabelle 1**

| Minimale Inhibitorische Konzentration des erfindungsgemäßen antifungischen Polypeptids gegenüber Pilzen in vitro (»g/ml) | |
|---|---|
| Trichophyton mentagrophytes | 125 |
| Trichophyton rubrum | 62,5 |
| Microsporum canis | 15,2 |
| Candida albicans | >> 125 |
| Aspergillus niger | 7,81 |
| Aspergillus tereus | 7,5 |
| Aspergillus fumigatus | 7,3 |
| Aspergillus nidulans | 7,4 |
| Aspergillus giganteus | 7,8 |
| Penicillium carylophilium | 7,9 |
| Fusarium aquaductum | 7,2 |
| Fusarium oxisporum | 7,3 |

### 5. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile Wirkstoff, 65 Gew.-Teile kaolinhaltigen Quarz als Inerstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.
c) Ein Granulat läßt sich herstellen aus 2 bis 15 Gew.-Teilen Wirkstoff und einem inerten Granulatträgermaterial wie Attapulgit, Bimsgranulat und/oder Quarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

### 6. Biologische Beispiele

Ca. 14 Tage alte Ackerbohnen der Sorten "Herz Freya" oder "Frank's Ackerperle" wurden mit dem erfindungsgemäßen Polypeptid gleichmäßig benetzt.

Nach Antrocknen des Spritzbelages wurden die Pflanzen mit einer Sporensuspension (1,5 Mio. Sporen/ml) von Botrytis cinerea (BCM- und Dicarboximid-sensitiver Stamm = Tabelle 2, BCM- und Dicarboximid-resistenter Stamm = Tabelle 3) inokuliert. Die Pflanzen wurden in einer Klimakammer bei 20-22°C und ca. 99 % rel. Luftfeuchte weiterkultiviert. Eine Infektion der Pflanzen äußert sich in der Bildung schwarzer Flecken auf Blättern und Stengeln. Die Auswertung der Versuche erfolgte ca. 1 Woche nach Inokulation.

Der Wirkungsgrad der Prüfsubstanzen wurde prozentual zur unbehandelten, infizierten Kontrolle bonitiert und ist in Tabelle 2 wiedergegeben.

**Tabelle 2**

| Verbindung gemäß Beispiel | Wirkungsgrad in % bei mg Wirkstoff (Polypeptid) pro 1 Liter Spritzbrühe | | | | |
|---|---|---|---|---|---|
| | 500 | 250 | 125 | 60 | 30 |
| Polypeptid | 100 | 100 | 100 | 99 | 85 |
| unbehandelte, infizierte Pflanzen | | | 0 | | |

**Tabelle 3**

| Verbindung gemaß Beispiel | Wirkungsgrad in % bei mg Wirkstoff (Polypeptid) pro 1 Liter Spritzbrühe | | | | |
|---|---|---|---|---|---|
| | 500 | 250 | 125 | 60 | 30 |
| Polypeptid | 100 | 100 | 100 | 98 | 85 |
| unbehandelte, infizierte Pflanzen | | | 0 | | |

### 7. Inokulation von transgenen Tomatenpflanzen mit Botrytis cinerea

Tomatenpflanzen des Wildtyps und transgene Pflanzen der gleichen Sorte wurden im 5-6 Blattstadium mit Konidiosporen von Botrytis cinerea inokuliert und anschließend für 3-4 Tage bei 20°C und 99 % rel. Luftfeuchte gehalten. Danach erfolgte die Auswertung des Versuches über Augenscheinbonitur; dabei wurde die mit Botrytis cinerea befallene Blattfläche (olivgraue Flecken und Welkesymptome) der transgenen Pflanzen im Vergleich zu den Wildtypen bewertet. Der Befallsgrad der Pflanzen ist aus Tab. 1 ersichtlich.

**Tabelle 4**

| Tomatenpflanzen | Botrytis cinerea-Befall in % befallener Blattfläche |
|---|---|
| Wildtyp | 90 - 100 |
| transgene Pflanzen | 30 - 60 |

### 8. Inokulation von transgenen Tabakpflanzen mit Sclerotinia sclerotiorum

Tabakpflanzen des Wildtyps und transgene Pflanzen der gleichen Sorte wurden im 4-Blattstadium mit Hyphenstücken (Suspension) von Sclerotinia sclerotiorum inokuliert und bei ca. 25°C und > 90 % relativer Luftfeuchte für ca. 3 Tage gehalten. Danach wurde die mit Sclerotinia sclerotiorum befallene Blattfläche (glasige Flecken, Blattfäulen, Myzelauflagerungen) der transgenen Versuchspflanzen im Vergleich zu den Wildtyp-Pflanzen bewertet. Der Befallsgrad der Pflanzen ist auf Tabelle 2 ersichtlich.

**Tabelle 5**

| Tabakpflanzen | Sclerotinia sclerotiorum-Befall in % befallener Blattfläche |
|---|---|
| Wildtyp | 100 |
| transgene Pflanzen | 30 - 55 |

## Patentansprüche

1. Polypeptid mit der Aminosäuresequenz

Ala-Thr-Tyr-Asn-Gly-Lys-Cys-Tyr-Lys-Lys-Asp-Asn-Ile-
Cys-Lys-Tyr-Lys-Ala-Gln-Ser-Gly-Lys-Thr-Ala-Ile-Cys-
Lys-Cys-Tyr-Val-Lys-Lys-Cys-Pro-Arg-Asp-Gly-Ala-Lys-
Cys-Glu-Phe-Asp-Ser-Tyr-Lys-Gly-Lys-Cys-Tyr-Cys.

2. Pflanzenzelle, die das Gen des antifungischen Peptids aus Aspergillus giganteus aus Anspruch 1 exprimiert.

3. Pflanze, enthaltend Zellen nach Anspruch 2.

4. Das Polypeptid aus Anspruch 1 exprimierende Pflanze, pflanzliches Gewebe oder Vermehrungsgut, gezüchtet aus einer Pflanzenzelle nach Anspruch 2.

5. Verfahren zur Herstellung der Pflanzenzelle, dadurch gekennzeichnet, daß das Gen des antifungischen Polypeptids aus Aspergillus giganteus in der Pflanzenzelle exprimiert wird.

6. Verfahren zur Herstellung des Polypeptids nach Anspruch 1, dadurch gekennzeichnet, daß Aspergillus giganteus kultiviert wird, vorzugsweise bei einer Temperatur von 18 bis 35°C, und das Polypeptid isoliert wird.

7. Mittel zur Inhibierung oder Abtötung von phytopathogenen Pilzen auf Pflanzen, gekennzeichnet durch einen Gehalt an dem Polypeptid nach Anspruch 1 und gegebenenfalls zusätzlich an einem anderen Fungizid oder einen weiteren Wirkstoff aus der Gruppe der Insektizide, Lockstoffe, Sterilantien, Akarazide, Nematizide oder Herbizide.

8. Verfahren zur Herstellung des Mittels gemäß Anspruch 7, dadurch gekennzeichnet, daß man das Polypeptid nach Anspruch 1 alleine oder zusammen mit dem anderen Fungizid oder dem weiteren Wirkstoff in eine geeignete Formulierung bringt.

9. Verfahren zum Schutz von Pflanzen gegen phytopathogene Pilze, dadurch gekennzeichnet, daß man die Pflanze, den Pflanzensamen oder die Anbauflächen mit einer wirksamen Menge des Polypeptids nach Anspruch 1 behandelt, vorzugsweise vor, nach oder gleichzeitig mit der Behandlung mit einem anderen Fungizid oder einem Insektizid, Lockstoff, Sterilans, Akarazid, Nematizid oder Herbizid.

10. Verwendung des Polypeptids nach Anspruch 1 als antifungisches Agens.

11. Verwendung des Polypeptids nach Ansspruch 1 in einem Verfahren nach Anspruch 9 als Agens gegen phytopathogene Pilze.

## Claims

1. A polypeptide with the amino acid sequence

Ala-Thr-Tyr-Asn-Gly-Lys-Cys-Tyr-Lys-Lys-Asp-Asn-Ile-
Cys-Lys-Tyr-Lys-Ala-Gln-Ser-Gly-Lys-Thr-Ala-Ile-Cys-
Lys-Cys-Tyr-Val-Lys-Lys-Cys-Pro-Arg-Asp-Gly-Ala-Lys-
Cys-Glu-Phe-Asp-Ser-Tyr-Lys-Gly-Lys-Cys-Tyr-Cys.

2. A plant cell which expresses the gene of the antifungal peptide from Aspergillus giganteus from Claim 1.

3. A plant containing cells according to Claim 2.

4. A plant, plant tissue or reproductive material expressing the polypeptide from Claim 1 and grown from a plant cell according to Claim 2.

5. A process for the production of the plant cell, characterized in that the gene of the antifungal polypeptide from Aspergillus giganteus is expressed in the plant cell.

6. A process for the production of the polypeptide according to Claim 1, characterized in that Aspergillus giganteus is cultivated, preferably at a temperature of 18 to 35°C, and the polypeptide is isolated.

7. A composition for inhibiting or destroying phytopathogenic fungi on plants, characterized in that it contains the polypeptide according to Claim 1 and additionally, if appropriate, another fungicide or another active ingredient from the group comprising insecticides, attractants, sterilizing agents, acaricides, nematicides or herbicides.

8. A process for the production of the composition according to Claim 7, characterized in that the polypeptide according to Claim 1 is incorporated into a suitable formulation, either by itself or together with the other fungicide or the other active ingredient.

9. A method of protecting plants from phytopathogenic fungi, characterized in that the plant, the plant seed or the cultivated areas are treated with an active amount of the polypeptide according to Claim 1, preferably before, after or at the same time as treatment with another fungicide or an insecticide, attractant, sterilizing agent, acaricide, nematicide or herbicide.

10. Use of the polypeptide according to Claim 1 as an antifungal agent.

11. Use of the polypeptide according to Claim 1 in a method according to Claim 9 as an agent against phytopathogenic fungi.

## Revendications

1. Polypeptide comportant la séquence des amino acides :

Ala-Thr-Tyr-Asn-Gly-Lys-Cys-Tyr-Lys-Lys-Asp-Asn-Ile-
Cys-Lys-Tyr-Lys-Ala-Gln-Ser-Gly-Lys-Thr-Ala-Ile-Cys-
Lys-Cys-Tyr-Val-Lys-Lys-Cys-Pro-Arg -Asp-Gly-Ala-Lys-
Cys-Glu-Phe-Asp-Ser-Tyr-Lys-Gly-Lys-Cys-Tyr-Cys.

2. Cellule végétale, qui exprime le gène du peptide antifongique ou fongicide provenant de Aspergillus giganteus, de la revendication 1.

3. Plante contenant des cellules selon la revendication 2.

4. Plante, tissu végétal ou matière de croissance exprimant le polypeptide de la revendication 1, que l'on a cultive à partir d'une cellule végétale selon la revendication 2.

5. Procédé de préparation de cellules végétales, caractérisées en ce qu'on provoque l'expression, dans les cellules de plantes, du gène du polypeptide antifongique provenant de Aspergillus giganteus.

6. Procédé de préparation du polypeptide selon la revendication 1, caractérisé en ce qu'on cultive Aspergillus giganteus, avantageusement à une température de 18 à 35°C, et l'on isole le polypeptide.

7. Produit pour inhiber ou détruire des champignons phytopathogènes sur des plantes, produit caractérisé par une teneur en le polypeptide selon la revendication 1, et éventuellement, en outre, en un autre fongicide ou en une autre substance active choisie dans l'ensemble formé par les insecticides, les appâts ou substances d'attraction, les stérilisants, les acaricides, les nématicides ou les herbicides.

8. Procédé de préparation du produit selon la revendication 7, caractérisé en ce qu'on met en une formulation convenable le polypeptide selon la revendication 1, seul ou avec l'autre fongicide ou avec l'autre substance active.

9. Procédé pour protéger des plantes contre des champignons phytopathogènes, caractérisé en ce qu'on traite la plante, les graines des plantes ou les surfaces de culture des plantes par une quantité efficace du polypeptide selon la revendication 1, avantageusement avant, après ou pendant le traitement avec un autre fongicide ou avec un insecticide, une substance d'appât, une matière stérilisante, un acaricide, un nématocide ou un herbicide.

10. Utilisation du polypeptide selon la revendication 1 comme agent antifongique ou fongicide.

11. Utilisation du polypeptide selon la revendication 1, dans un procédé selon la revendication 9, comme agent de lutte contre des champignons phytopathogènes.
